# EUROPÄISCHE PATENTSCHRIFT

(11) **EP 0 724 426 B1**
(45) Veröffentlichungstag und Bekanntmachung des Hinweises auf die Patenterteilung: **20.05.1998**
(21) Anmeldenummer: 94929563.8
(22) Anmeldetag: 20.10.1994
(51) Int. Cl.: A61K 7/50, A61K 7/48

(54) **HAUTREINIGUNGSMITTEL, VERFAHREN ZU IHRER HERSTELLUNG UND IHRE VERWENDUNG**
SKIN-CLEANSING AGENTS, METHODS OF PREPARING THEM AND THEIR USE
PRODUITS DE NETTOYAGE DE LA PEAU, LEURS PROCEDES DE PREPARATION ET LEUR UTILISATION

(30) Priorität: 21.10.1993 DE 4335933
(43) Veröffentlichungstag der Anmeldung: 07.08.1996
(73) Patentinhaber: Stockhausen GmbH & Co. KG, 47805 Krefeld (DE)
(72) Erfinder: DANIEL, Günter, D-47803 Krefeld (DE); ROSENBERGER, Volker, D-41564 Kaarst (DE); BRÜCHER, Beatrice, D-47807 Krefeld (DE)
(74) Vertreter: Klöpsch, Gerald, Dr.-Ing. Patentanwalt
(86) Internationale Anmeldenummer: EP9403448
(87) Internationale Veröffentlichungsnummer: WO9511005

(56) Entgegenhaltungen:
- EP-A- 0 158 108
- EP-A- 0 513 832
- WO-A-89/03669
- BE-A- 693 350
- US-A- 4 344 446

## Beschreibung

Die Erfindung betrifft Hautreinigungsmittel mit verbesserter Reinigungswirkung und hautschonendem Verhalten, Verfahren zu ihrer Herstellung und die Verwendung der Mittel als fließfähige Handwaschmittel bzw. Grobhandreiniger.

Handreiniger zur Entfernung von insbesondere stark haftenden Hautverunreinigungen werden bereits im industriellen Bereich verwendet. Die Patentanmeldung WO 91/14 420 beschreibt Reinigungsmittel, die neben anionischen und/oder nichtionogenen Tensiden, übliche Abrasiva, organische Lösemittel in Form von Carbonsäureestern, insbesondere bestimmte Acetate, wie vorzugsweise n-Butoxy-2-ethoxy-ethyl-acetat sowie Fettalkanolamide, -polyalkanolamide, deren Etylenoxid und/oder Propylenoxid-anlagerungsprodukte und Fettsäuremonoglyceride als Rückfettungsmittel enthalten.

Ein anderes bevorzugtes, zusätzlich verwendetes Lösemittel in diesen Handreinigungsmitteln ist Limonen, von dem eine dermale Unverträglichkeit, verbunden mit einem Sensibilisierungsrisiko, bekannt ist. Zur rationellen Herstellung der Mittel nach WO 91/14 420 ist es erforderlich, nach dem Vermischen der Rohstoffe die Mischung auf Temperaturen oberhalb 50° C zu erwärmen, wozu zusätzliche Heizenergie verbraucht und die Produktionszeit verlängert wird.

Weiterhin sind Hautreiniger bekannt, die als Lösemittelkomponente natürliche Öle, wie Oliven-Öl, Jojoba-Öl, Macadamia-Nuts-Öl und Traubenkernöl enthalten. Diese Reinigungsmittel sind wegen der Wechselwirkung zwischen den Öl- und Tensidanteilen und der geringen schmutzlösenden Wirkung des natürlichen Öls in ihrer Reinigungskraft eingeschränkt.

In der Kosmetik ist weiterhin die Verwendung von speziellen Estern der Adipinsäure bekannt. So wird Di-n-butyladipat wegen seines schwach fettenden Charakters in Tagescremes und flüssigen Emulsionen sowie in Haarsprays und -festigern als weichmachende Komponente bzw. Überfettungsmittel eingesetzt.

Die Patentschrift GB 1,106,945 beschreibt wässrige Shampoo-Präparationen zur Haarreinigung, die als Lösemittel für Fette Alkylester der dibasischen Carbonsäuren, Phthalsäure bzw. Adipinsäure enthalten, wobei bei der Haarreinigung und Haarkonditionierung besonders gute Ergebnisse mit Dimethylphthalat erhalten werden, ohne daß jedoch ein Hinweis auf eine Hautreinigung, insbesondere eine Reinigung von durch Fremdstoffe grob verschmutzter Haut erfolgt.

Die EP 229 616 A2 beschreibt Badezusätze, die als sich auf der Haut ablagernde Ölkomponente Diisopropyladipat enthalten.

Die EP 513 832 A1 beschreibt pharmazeutische Produkte, die zur Verbesserung bzw. Kontrolle der Hautpenetration eines therapeutischen Mittels Dibutyladipat oder eine Kombination Dibutyladipat und Isopropylmyristat enthalten.

Di-isopropyladipat wird weiterhin als Gleitmittel in alkoholischen Lotionen, als Weichmacher in Haar-Aerosolen und als Fettfaktor in Haarwässer sowie als Lösevermittler für Riechstoffe verwendet.

Die PCT-Anmeldung WO 92/09 265 beschreibt eine lösemittelhaltige Handwaschpaste, die als Lösemittel DBE 2 Dimethyladipat, Dimethylglutarat und Dimethylsuccinat, insgesamt in einer Menge von 54 Teilen auf 100 Teile Reinigungsmittel, sowie iso-Octylstearat als zusätzlich notwendiges Rückfettungsmittel und gebleichtes Kernschalenmehl als hautschonendes Abrasivum enthält.

In der EP 0166522 A2 wird eine kosmetische Zubereitung zur Entfernung von Nagellack beschrieben, die zugleich hautreinigende Eigenschaften aufweist und die als Lösemittel für den Nagellack Ester von Dicarbonsäuren, bevorzugt Diester oder Ester von Diolen, bevorzugt Dioldiester, enthalten. Als Dicarbonsäureester werden die Diethylester der Dicarbonsäuren mit bis zu 6 C-Atomen bevorzugt, als Diolester Diacetate von Diolen mit 2 bis 6 C-Atomen. Die Zubereitungen enthalten ferner Lanolin oder andere rückfettende Komponenten und kein Reibmittel.

Die Verwendung der bekannten Lösemittel in Hautreinigungsmitteln macht es so bisher erforderlich, deren intensive Einwirkung auf die Haut durch die Wirkung von Rückfettungsmitteln zu kompensieren. Allerdings wird durch die rückfettenden Komponenten auch die Reinigungswirkung der Tenside geringfügig beeinträchtigt.

Es bestand daher die Aufgabe, Hautreinigungsmittel und ein Verfahren zu ihrer Herstellung bereitzustellen, deren Lösemittel eine vergleichbare oder verbesserte Reinigungswirkung und damit eine reinigungsverstärkende Wirkung sowie eine verbesserte dermatologische Verträglichkeit aufweisen. Es bestand weiterhin die Aufgabe, ein Herstellungsverfahren für Hautreinigungsmittel zu finden, bei dem die Erwarmung der Rohstoffmischung ausgelassen werden kann.

Diese Aufgaben wurden durch Hautreinigungmittel gelöst, die als Lösemittel ausschließlich Di-n-butyladipat und/oder Di-isopropyladipat und daneben als Hauptkomponenten waschaktive Substanzen, Komponenten zur Verbesserung der Konsistenz, des Aussehens und Geruchs und der Lagerstabilität sowie wenigstens ein Abrasivum und Wasser enthalten und deren Herstellung zur Verbesserung der Reinigungswirkung und der Herstellungsbedingungen unter Verwendung bestimmter Fettalkohole erfolgen kann.

Gegenstand der Erfindung sind wasserhaltige, flüssige, pasten- oder cremeförmige Hautreinigungsmittel zur Entfernung von stark haftenden Verschmutzungen, die anionische und/oder amphotere und/oder nichtionogene Tenside als waschaktive Substanzen, Verdickungsmittel, wenigstens ein Abrasivum sowie gegebenenfalls Hilfsstoffe zur Regulierung der Konsistenz von Aussehen, Geruch und Stabilität wie Pigmente, Duftstoffe, Stabilisatoren und Konservierungsmittel enthalten und die dadurch gekennzeichnet sind, daß sie als ausschließliche Lösungsmittel Di-n-butyladipat und/oder Di-isopropyladipat enthalten.

Als waschaktive Substanzen werden bekannte Detergentien, insbesondere anionische und/oder amphotere und/oder nichtionoge Tenside, z. B. Alkylsulfate, -sulfonate, -Alkylethersulfate und Fettalkohol-polyglykolsulfo-succinate, die überwiegend in Form ihrer Na-Salze eingesetzt werden, sowie die bekannten nichtionogenen Tenside auf der Basis von Anlagerungsprodukten von Alkylenoxiden an Alkanolen, Alkansäuren, die auch endverschlossen sein können, sowie vorzugsweise Alkylpolyglykoside verwendet.

Vorzugsweise verwendbare Tenside sind Na-laurylethersulfat, Ricinusölsulfonat, Di-Nalaurylpolyglykolethersulfosuccinat, Cocoamidopropylbetain und Alkylpolyglycoside mit einem Alkylrest aus C₈ - C₁₆ -, vorzugsweise C₈ bis C₁₀ und einem Oligoglycosidrest mit einem Oligomerisierungsgrad von 1,1 - 1,7, vorzugsweise 1,2 - 1,3.

Der Anteil der waschaktiven Substanzen beträgt 5 - 40 Gew.%, vorzugsweise 10 - 30 Gew.%, bezogen auf das erfindungsgemäße Hautreinigungsmittel.

Der Anteil der in den erfindungsgemäßen Mitteln enthaltenen Ester Di-n-butyladipat und/oder Di-isopropyladipat beträgt 1 - 25 Gew.%, vorzugsweise 5 bis 20 Gew.%, und besonders bevorzugt 10 - 20 Gew.%.

Bevorzugt zu verwendende Abrasiva sind die gebleichten Kernschalenmehle nach WO 92/09265, insbesondere gebleichtes Walnußschalen- oder Olivenkernmehl. Der Anteil des Abrasivum im erfindungsgemäßen Mittel beträgt 1 - 30 Gew.%, bevorzugt 10 - 20 Gew.%.

Die erfindungsgemäßen Mittel enthalten weiterhin viskositätsbildende Mittel wie organophile und/oder hydrophile Schichtsilikate wie Bentonite, Polysaccharide wie Cellulose und Guarmehl sowie modifizierte Polysaccharide, wie die Celluloseether Carboxylmethylcellulose und Hydroxyethylcellulose und Xanthan gum sowie anorganische Elektrolyte, wie NaCl oder MgSO₄. Sie enthalten weiterhin farbgebende Pigmente, wie TiO₂, Stabilisatoren wie Propylencarbonat, pH-Regulatoren, Duftstoffe und Konservierungsmittel. Der Wassergehalt der Hautreinigungsmittel beträgt 10 - 50 Gew.%, vorzugsweise 20 - 40 Gew.%.

Bei der Herstellung der erfindungsgemäßen Hautreinigungsmittel kann auf die Verwendung von rückfettenden Komonenten verzichtet werden.

Vorzugsweise werden die Hautreinigungsmittel als flüssige oder cremige Mittel oder als fließfähige viskose Pasten erhalten.

In einer weiteren bevorzugten Ausführungsform enthalten die erfindungsgemäßen Hautreinigungsmittel zusätzlich wenigstens einen Fettalkohol der allgemeinen Formel R-OH, wobei R für einen Alkylrest mit C₈ bis C₁₆ steht, vorzugsweise C₈ - C₁₂. Die Menge an Fettalkohol im Mittel beträgt 0,1 - 10,0 Gew.%, vorzugsweise 0,1 - 5,0 Gew.%.

Die angegebenen Rohstoffkomponenten können üblicherweise auch unter Verwendung von Kombinationen einer Rohstoffart, also als Mischungen verschiedener Tenside, Viskositätsbildner, Abrasiva und Stabilisatoren etc. verwendet werden.

Die Herstellung der fließfähigen Hautreinigungsmittel erfolgt mittels bekannter Vorrichtungen im Batch- oder Continue-Verfahren. Geeignete Vorrichtungen sind temperierbare Kessel mit Rührwerk, Mischer und Extruder, wie sie beispielsweise in "Surfactans in Consumer Products", J. Falbe, Springer Verlag 1987, S. 399 ff. beschrieben werden.

Die Erfindung betrifft ferner ein Verfahren zur Herstellung von Hautreinigungsmitteln, bei denen die Komponenten unter Rühren wie üblich, ggf auch in Teilmengen der Rohstoffe zusammengegeben werden, wobei vorzugsweise die Adipinester-Lösemittel vorgelegt, danach Pigment, Viskosität bildende Mittel/Verdickungsmittel und eine Teilmenge des Tensids zugegeben werden, diese Mischung erwärmt und vorzugsweise auf eine Temperatur bis zu 70° C, bevorzugt 40 - 60 °C, eingestellt wird, worauf anschließend die Zugabe der restlichen Rohstoffmengen, insbesondere Tensidmengen, des anorganischen Elektrolyten, des pH-Regulators und Parfumöls, des Konservierungsmittels sowie des Abrasivums erfolgt. Die homogene Mischung wird danach abgekühlt und in übliche Behälter wie Kanister, Pumpgefäße wie Spender, Dosen und Tuben abgefüllt.

Nach einer erfindungsgemäßen speziellen Herstellungsweise werden zur Herstellung der Mittel Fettalkohole als Konsistenzregulatoren zugesetzt. Es wurde überraschenderweise gefunden, daß der Zusatz des Fettalkohols die Mischung der Rohstoffe, beispielsweise in der bereits angegebenen Reihenfolge zu homogenen Produkten bei Raumtemperatur, d. h. bei 5 - 30 °C gestattet, ohne daß die Rohstoffmischung erwärmt zu werden braucht. Das Fortfallen der Erwärmungsphase und die damit verbundene Energie- und Zeitersparnis bieten verfahrenstechnische Vorteile.

Der Fettalkoholzusatz wirkt sich weiterhin günstig auf die Verwendung der Hautreinigungsmittel aus, da beispielsweise die Reinigungswirkung erhöht wird.

Die erfindungsgemäßen Hautreinigungsmittel sind besonders zur Entfernung von stark auf der Haut haltenden Grobverschmutzungen, wie beispielsweise Fette, Öle und andere Schmierstoffe, Farben, Lacke, Teer, Graphit, Ruß, Farbpigmente und ähnliche Stoffe, geeignet, wie sie im industriellen und öffentlichen Bereich, im Handwerk oder in der Landwirtschaft und im Haushalt vorkommen.

Die Verwendung der erfindungsgemäßen Hautreinigungsmittel erfolgt, indem zunächst bevorzugt das Reinigungsmittel auf der Haut ohne oder mit einer geringen Wassermenge verteilt wird, worauf die Reinigung mit Wasser fortgesetzt und unter Abspülen mit Wasser beendet wird. Die geringe Wassermenge ergibt sich durch eine Benetzung oder durch Anfeuchten der verschmutzten Hautflächen mit Wasser oder wässrigen Flüssigkeiten. Sie kann z. B. bei verschmutzten Händen ca. 1 - 3 ml Wasser pro Handfläche betragen.

Beim Waschvorgang lassen sich die erfindungsgemäßen Mittel leicht auf der Haut verteilen. Sie zeigen ein gutes Anschäumvermögen und eine gegenüber herkömmlichen Produkten verbesserte Reinigungswirkung, wobei die Verschmutzungen mit Wasser mühelos von der Haut entfernt werden. Neben der guten Schmutzentfernung besitzen die erfindungsgemäßen Mittel eine verbesserte dermatologische Verträglichkeit.

Durch die Verwendung geeigneter Rohstoffe unterliegen die Mittel dem biologischen Abbauprozeß und sind daher ebenfalls durch eine gute Umweltverträglichkeit gekennzeichnet.

Die Erfindung wird durch die folgende Beispiele erläutert.

| Rezeptur I | |
|---|---|
| Di-n-butyladipat | 10,00 Gew.-% |
| Titandioxid | 1,00 Gew.-% |
| Organophiler Bentonit | 2,10 Gew.-% |
| Carboxymethylcellulose | 1,20 Gew.-% |
| Ricinusölsulfonat (70%ig) | 10,53 Gew.-% |
| Propylencarbonat | 0,50 Gew.-% |
| Natriumfettalkoholethersulfat (C₁₂ - C₁₅) 28%ig | 47,00 Gew.-% |
| Wasser | 0,19 Gew.-% |
| Di-natrium-laurylpolyglykolether-sulfosuccinat (32 - 35%ig) | 10,00 Gew.-% |
| Siedesalz | 2,00 Gew.-% |
| Zitronensäure | 0,20 Gew.-% |
| Parfumöl | 0,20 Gew.-% |
| Konservierungsmittel | 0,08 Gew.-% |
| Walnußschalenmehl, gebleicht | 15,00 Gew.-% |
| | 100,00 Gew.-% |

| Rezeptur II | |
|---|---|
| Di-isopropyladipat | 10,00 Gew.-% |
| Titandioxid | 1,00 Gew.-% |
| Organophiler Bentonit | 2,00 Gew.-% |
| Hydrophiler Bentonit | 3,00 Gew.-% |
| Alkylpolyglycosid (C₈ - C₁₀) 55%ig | 20,00 Gew.-% |
| Stadtwasser | 1,89 Gew.-% |
| Zitronensäure | 0,23 Gew.-% |
| Konservierungsmittel | 0,08 Gew.-% |
| Siedesalz | 2,50 Gew.-% |
| Natriumfettalkoholethersulfat (C₁₂ - C₁₅), 28%ig | 44,00 Gew.-% |
| Walnußschalenmehl, gebleicht | 15,00 Gew.-% |
| Parfumöl | 0,30 Gew.-% |
| | 100,00 Gew.-% |

| Rezeptur III | |
|---|---|
| Lösungsmittel a bis g*) | 10,00 Gew.-% |
| Titandioxid | 1,00 Gew.-% |
| Hydrophiler Bentonit | 1,00 Gew.-% |
| Organophiler Bentonit | 4,00 Gew.-% |
| Alkylpolyglycosid (C₈ - C₁₀) 55%ig | 20,90 Gew.-% |
| Fettalkohol (C₈ - C₁₂) | 1,50 Gew.-% |
| Stadtwasser | 2,47 Gew.-% |
| Konservierungsmittel | 0,08 Gew.-% |
| Parfumöl | 0,30 Gew.-% |
| Zitronensäure | 0,15 Gew.-% |
| Siedesalz | 2,50 Gew.-% |
| Natriumfettalkoholethersulfat (C₁₂ - C₁₅) 28%ig | 44,00 Gew.-% |
| Walnußschalenmehl, gebleicht | 13,00 Gew.-% |
| | 100,00 Gew.-% |

| | |
|---|---|
| *) IIIa: Di-n-butyladipat IIIb: ohne Lösungsmittel IIIc: n-Paraffin IIId: Traubenkernöl IIIe: DBE 2 IIIf: Di-isopropyladipat IIIg: Di-n-butyladipat, ohne Fettalkohol (C₈ - C₁₂) | |

| Rezeptur IV | |
|---|---|
| Di-n-butyladipat | 10,00 Gew.-% |
| Titandioxid | 1,00 Gew.-% |
| Hydrophiler Bentonit | 1,00 Gew.-% |
| Organophiler Bentonit | 3,00 Gew.-% |
| Alkylpolyglycosid (C₈ - C₁₀) 55%ig | 20,00 Gew.-% |
| Fettalkohol (C₈ - C₁₂) | 4,00 Gew.-% |
| Stadtwasser | 0,97 Gew.-% |
| Konservierungsmittel | 0,08 Gew.-% |
| Parfumöl | 0,30 Gew.-% |
| Zitronensäure | 0,15 Gew.-% |
| Siedesalz | 2,50 Gew.-% |
| Natriumfettalkoholethersulfat (C₁₂ - C₁₅) 28%ig | 44,00 Gew.-% |
| Walnußschalenmehl, gebleicht | 13,00 Gew.-% |
| | 100,00 Gew.-% |

| Rezeptur V | |
|---|---|
| Di-n-butyladipat | 7,00 Gew.-% |
| Titandioxid | 1,00 Gew.-% |
| Hydrophiler Bentonit | 1,00 Gew.-% |
| Organophiler Bentonit | 2,50 Gew.-% |
| Alkylpolyglycosid (C₈-C₁₀) 55%ig | 20,00 Gew.-% |
| Fettalkohol (C₈-C₁₂) | 8,00 Gew.-% |
| Stadtwasser | 0,47 Gew.-% |
| Konservierungsmittel | 0,08 Gew.-% |
| Parfumöl | 0,30 Gew.-% |
| Zitronensäure | 0,15 Gew.-% |
| Siedesalz | 0,50 Gew.-% |
| Natriumfettalkoholethersulfat (C₁₂-C₁₅) 28%ig | 44,00 Gew.-% |
| Walnußschalenmehl, gebleicht | 15,00 Gew.-% |
| | 100,00 Gew.-% |

Die Reinigungswirkung der Formulierungen wurde im Handwaschtest nach der Methodenbeschreibung in DE 27 36 970 A1 geprüft. Dabei wurde festgestellt, daß die Mittel gegen einen Modellschmutz, hauptsächlich bestehend aus Öl, Wachs, Vaseline, Graphit, Flammruß und Eisenoxid sowie gegen einen Modellack aus Kunstharz, Fischöl und einem Farbpigment, deutlich verbesserte Reinigungseffekte zeigen.

Die Hautverträglichkeit wurde im Duhring-Kammer-Test [Frosch, P.J., Kligman, A.M.: The Duhring chamber and improved technique for epicutaneous testing of irritant and allergic reactions. Contact Derm. 5, 73 - 81 (1979); Frosch P.J, Kligman, A.M.: The soap chamber test, a new method for assessing the irritancy of soaps. J. Am. Acad. Derm. 1, 35 - 41 (1979)] durchgeführt. Die Ergebnisse weisen aus, daß die dermale Verträglichkeit der erfindungsgemäßen Mitteln gegenüber handelsüblichen, bekannten Produkten ebenfalls verbessert ist.

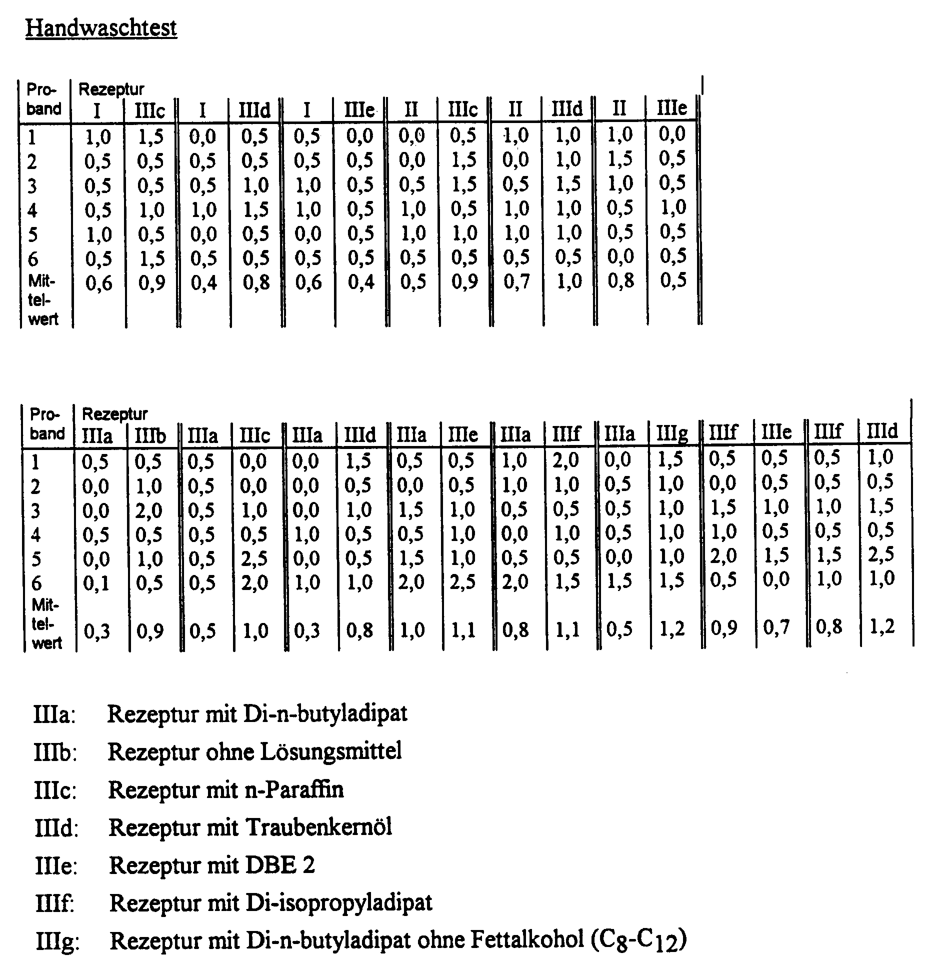

| | |
|---|---|
| Prüfproduktmenge: | 1,2 g |
| Schmutzmenge: | 0,5 g |

### Ergebnis:

Rezeptur I mit Di-n-buryladipat und Rezeptur II mit Di-isopropyladipat besitzen jeweils eine bessere Reinigungswirkung als IIIc und IIId jedoch eine etwas schlechtere Wirkung als IIIe.

Die durchgeführten Waschversuche zeigen deutlich die Überlegenheit der Di-n-butyladipat und Fettalkohol (C₈-C₁₂)-haltigen Rezeptur IIIa gegenüber solchen ohne Lösungsmittel (IIIb), mit Di-n-butyladipat, (IIIg), mit n-Paraffin IIIc und Traubenkernöl (IIId).

Gegenüber den Rezepturen, die DBE 2 (Gemisch aus Dimethyladipat, Dimethylsinccinat, Dimethylglutarat) IIIe und Di-isoproppyladipat IIIf enthalten, besitzt IIIa eine vergleichbare bzw. bessere Reinigungswirkung.

Weiterhin besitzt die Rezeptur mit Di-isopropyladipat IIIf eine bessere Reinigungswirkung als diejenige mit Traubenkernöl IIId, jedoch eine etwas schlechtere als die Formulierung mit DBE 2 IIIe.

| Duhring-Kammer-Test | | | | | | | | |
|---|---|---|---|---|---|---|---|---|
| | Wasser | NaLS 0,5%ig | Rezeptur I | II | IV | V | IIIb | |
| A-Wert | 25,1 | 4,3 | 16,1 | 14,4 | 14,8 | 15,3 | 14,2 | |

| | Wasser | NaLS 0,5%ig | Rezeptur IIIa | IIIc | IIId | IIIe | IIIf | IIIg |
|---|---|---|---|---|---|---|---|---|
| A-Wert | 25,1 | 4,3 | 15,2 | 5,0 | 17,1 | 8,2 | 14,7 | 14,5 |
| IIIa: Rezeptur mit Di-n-butyladipat IIIb: Rezeptur ohne Lösungsmittel IIIc: Rezeptur mit n-Paraffin IIId: Rezeptur mit Traubenkernöl IIIe: Rezeptur mit DBE 2 IIIf: Rezeptur mit Di-isopropyladipat IIIg: Rezeptur mit Di-n-butyladipat ohne Fettalkohol (C₈-C₁₂) | | | | | | | | |

| | |
|---|---|
| Prüfproduktmenge: | ca. 200 mg |
| Testzeitraum: | 3 Wochen |
| Applikationsintervalle: | 1. Tag 2 Stunden |
| | 2. Tag 4 Stunden |
| | 3. Tag 6 Stunden |
| | 4. Tag 6 Stunden |
| | 5. Tag ca. 5 Stunden |
| Anzahl der Probanden: | 25 |

### Ergebnis:

Der durchgeführte Duhring-Kammer-Test belegt die für diese Produktklasse gute dermale Verträglichkeit der erfindungsgemäßen Rezepturen I, II, IV, IIIa, IIIf und IIIg. Im Vergleich dazu zeigen die Rezepturen mit n-Paraffin (IIIc) und DBE 2 (IIIe) eine deutlich schlechtere Hautverträglichkeit.

## Patentansprüche

1. Wasserhaltige, flüssige, pasten- oder cremeförmige Hautreinigungsmittel zur Entfernung von stark haftenden Verschmutzungen, enthaltend anionische und/oder amphotere und/oder nichtionogene Tenside als waschaktive Substanzen, Viskositätsbildner, wenigstens ein Abrasivum und ggf Hilfsstoffe zur Regulierung der Konsistenz, des Aussehens, Geruchs und der Stabilität wie Pigmente, Duftstoffe, Stabilisatoren und Konservierungsmittel, dadurch gekennzeichnet, daß sie als Lösemittel ausschließlich Di-n-butyladipat und/oder Di-isopropyladipat enthalten.

2. Wasserhaltige, flüssige, pasten- oder cremeförmige Hautreinigungsmittel nach Anspruch 1, dadurch gekennzeichnet, daß sie wenigstens einen Fettalkohol der allgemeinen Formel R-OH enthalten, wobei R ein Alkylrest mit C₈ bis C₁₆ vorzugsweise C₈ bis C₁₂ ist.

3. Wasserhaltige, flüssige, pasten- oder cremeförmige Hautreinigungsmittel nach einem der Ansprüche 1-2, dadurch gekennzeichnet, daß der Gehalt an Lösemittel 1-25, vorzugsweise 5-20 Gew.-%, bezogen auf das Hautreinigungsmittel, beträgt.

4. Wasserhaltige, flüssige, pasten- oder cremeförmige Hautreinigungsmittel nach einem der Ansprüche 1 bis 3, dadurch gekennzeichnet, daß der Gehalt an Fettalkohol 0,1 bis 10,0 Gew.-%, vorzugsweise 0,1 bis 5,0 Gew.-%, bezogen auf das Hautreinigungsmittel, beträgt.

5. Wasserhaltige, flüssige, pasten- oder cremeförmige Hautreinigungsmittel nach einem der Ansprüche 1 bis 4, dadurch gekennzeichnet, daß sie als nichtionogene Tenside Alkylpolyglycoside mit einem Alkylrest aus C₈ bis C₁₆, vorzugswesie C₈ bis C₁₀, und einem Oligoglycosidrest mit einem Oligomerisierungsgrad von 1,1 bis 1,7, vorzugsweise 1,2 bis 1,3, Natriumfettalkoholethersulfat und gebleichtes Walnußschalenmehl enthalten.

6. Wasserhaltige, flüssige, pasten- oder cremeförmige Hautreinigungsmittel nach einem der Ansprüche 1 - 5, dadurch gekennzeichnet, daß der Gehalt an waschaktiven Substanzen 5 - 40, vorzugsweise 10 - 30 Gew.-%, bezogen auf das Hautreinigungsmittel, beträgt.

7. Wasserhaltige, flüssige, pasten- oder cremeförmige Hautreinigungsmittel nach einem der Ansprüche 1 - 6, dadurch gekennzeichnet, daß der Gehalt an Abrasivum 1 - 30, bevorzugt 10 - 20 Gew.-%, beträgt.

8. Wasserhaltige, flüssige, pasten- oder cremeförmige Hautreinigungsmittel nach einem der Ansprüche 1 - 7, dadurch gekennzeichnet, daß der Wassergehalt 10 - 50, vorzugsweise 20 - 40 Gew.-%, beträgt.

9. Verfahren zur Herstellung der Hautreinigungsmittel nach einem der Ansprüche 1 bis 8, dadurch gekennzeichnet, daß zunächst das Lösemittel vorgelegt, danach Pigment, Viskosität bildende Mittel/Verdickungsmittel und eine Teilmenge des Tensids zugegeben werden, die so erhaltene Mischung auf eine Temperatur bis zum 70° C, bevorzugt 40 - 60° C, erwärmt wird, worauf anschließend die restlichen Komponentenmengen, insbesondere Tensidmengen, der anorganische Elektrolyt, der pH-Regulator, das Parfümöl, das Konservierungsmittel sowie das Abrasivum zugesetzt werden und homogenisiert und schließlich abgekühlt wird.

10. Verfahren nach Anspruch 9, dadurch gekennzeichnet, daß bei Verwendung eines Fettalkohols als Komponente das Vermischen der Komponenten und Homogenisieren ohne Erwärmung durchgeführt wird.

11. Verwendung der Hautreinigungsmittel nach einem der Ansprüche 1 bis 8, dadurch gekennzeichnet, daß beim Reinigungsvorgang das Reinigungsmittel, vorzugsweise zunächst ohne Wasserzusatz oder mit einer geringen Wassermenge, auf der Haut verteilt und der Reinigungsvorgang unter Abspülen mit Wasser abgeschlossen wird.

## Claims

1. Hydrous, liquid, pasty or creamy skin cleansers for removing strongly adhering dirt, which contain anionic and/or amphoteric and/or non-ionogenic surfactants as detergent substances, viscosity-forming agents, at least one abrasive and optionally, adjuvants for controlling consistency, aspect, odor and stability, such as pigments, perfumes, stabilizers and preservatives, characterized in that they only contain di-n-butyl adipate and/or diisopropyl adipate as solvent.

2. The hydrous, liquid, pasty or creamy skin cleansers according to claim 1, characterized in that they include at least one fatty alcohol of general formula R-OH wherein R is an alkyl residue of from C₈ to C₁₆, preferably from C₈ to C₁₂.

3. The hydrous, liquid, pasty or creamy skin cleansers according to one of claims 1-2, characterized in that the solvent content is 1-25, preferably 5-20% by weight, relative to the skin cleanser.

4. The hydrous, liquid, pasty or creamy skin cleansers according to one of claims 1-3, characterized in that the fatty alcohol content is from 0.1 to 10.0% by weight, preferably from 0.1 to 5.0% by weight, relative to the skin cleanser.

5. The hydrous, liquid, pasty or creamy skin cleansers according to one of claims 1-4, characterized in that they contain alkyl polyglycosides having an alkyl residue of from C₈ to C₁₆, preferably from C₈ to C₁₀, and an oligoglycoside residue with an oligomerization degree of from 1.1 to 1.7, preferably from 1.2 to 1.3, sodium fatty alcohol ether sulfate and bleached walnut shell meal as non-ionogenic surfactants.

6. The hydrous, liquid, pasty or creamy skin cleansers according to one of claims 1-5, characterized in that the content of detergent substances is 5-40, preferably 10-30% by weight, relative to the skin cleanser.

7. The hydrous, liquid, pasty or creamy skin cleansers according to one of claims 1-6, characterized in that the content of abrasive is 1-30, preferably 10-20% by weight.

8. The hydrous, liquid, pasty or creamy skin cleansers according to one of claims 1-7, characterized in that the water content is 10-50, preferably 20-40% by weight.

9. A process for producing the skin cleansers according to one of claims 1-8, characterized in that the solvent is charged initially, then the pigment, viscosity-forming agent/thickening agent, and part of the surfactant are added, the mixture thus obtained is heated at a temperature of up to 70°C, preferably 40-60°C, followed by addition of the remaining amounts of components, particularly the amounts of surfactants, the inorganic electrolyte, the pH regulator, the perfume oil, the preservative, and the abrasive, and homogenization and eventual cooling.

10. The process according to claim 9, characterized in that when using a fatty alcohol as component, mixing of the components and homogenization is effected without heating.

11. Use of the skin cleansers according to one of claims 1-8, characterized in that during the cleansing procedure, the cleanser is distributed over the skin, preferably with no water or a small amount of water added at first, and the cleansing procedure is completed by rinsing with water.

## Revendications

1. Agents de nettoyage de la peau, sous forme de pâte ou pommade ou crème, fluide, contenant de l'eau, en vue d'éliminer les souillures fortement adhérentes, contenant des agents tensioactifs anioniques et/ou amphotères et/ou non ionogènes, à titre de substances détergentes, des agents conférant de la viscosité, au moins un abrasif et, éventuellement, des adjuvants de régulation de la consistance, de l'aspect, de l'odeur et de la stabilité, comme des pigments, des parfums, des stabilisateurs et des conservateurs, caractérisés en ce qu'ils contiennent exclusivement de l'adipate de di-n-butyle et/ou de l'adipate de diisopropyle, à titre de solvants.

2. Agents de nettoyage de la peau, sous forme de pâte ou pommade ou crème, fluide, contenant de l'eau suivant la revendication 1, caractérisés en ce qu'ils contiennent au moins un alcool gras de la formule générale R-OH, dans laquelle R représente un radical alkyle en C₈ à C₁₆, de préférence, en C₈ à C₁₂.

3. Agents de nettoyage de la peau, sous forme de pâte ou pommade ou crème, fluide, contenant de l'eau suivant l'une quelconque des revendications 1 et 2, caractérisés en ce que la teneur en solvants varie de 1 à 25% en poids, de préférence, 5 à 20% en poids, par rapport à l'agent de nettoyage de la peau.

4. Agents de nettoyage de la peau, sous forme de pâte ou pommade ou crème, fluide, contenant de l'eau suivant l'une quelconque des revendications 1 à 3, caractérisés en ce que la teneur en alcool gras varie de 0,1 à 10,0% en poids, de préférence, de 0,1 à 5,0% en poids, par rapport aux agents de nettoyage de la peau.

5. Agents de nettoyage de la peau, sous forme de pâte ou pommade ou crème, fluide, contenant de l'eau suivant l'une quelconque des revendications 1 à 4, caractérisés en ce qu'ils contiennent, à titre d'agents tensioactifs non ionogènes, des alkylpolyglycosides avec un radical alkyle en C₈ à C₁₆, de préférence, en C₈ à C₁₀ et un reste d'oligoglycoside avec un degré d'oligomérisation de 1,1 à 1,7, de préférence, de 1,2 à 1,3, un éthersulfate d'alcool gras sodique et de la farine de noix blanchie.

6. Agents de nettoyage de la peau, sous forme de pâte ou pommade ou crème, fluide, contenant de l'eau suivant l'une quelconque des revendications 1 à 5, caractérisés en ce que la teneur en substances détergentes varie de 5 à 40% en poids, de préférence, de 10 à 30% en poids, par rapport aux agents de nettoyage de la peau.

7. Agents de nettoyage de la peau, sous forme de pâte ou pommade ou crème, fluide, contenant de l'eau suivant l'une quelconque des revendications 1 à 6, caractérisés en ce que la teneur en abrasif varie de 1 à 30% en poids, de préférence, 10 à 20% en poids.

8. Agents de nettoyage de la peau, sous forme de pâte ou pommade ou crème, fluide, contenant de l'eau suivant l'une quelconque des revendications 1 à 7, caractérisés en ce que la teneur en eau varie de 10 à 50% en poids, de préférence, de 20 à 40% en poids.

9. Procédé de préparation des agents de nettoyage de la peau suivant l'une quelconque des revendications 1 à 8, caractérisé en ce que l'on prend au préalable le solvant, puis on y ajoute le pigment, des agents conférant de la viscosité/agents épaississants et une proportion partielle de l'agent tensioactif, on chauffe le mélange ainsi obtenu jusqu'à une température s'élevant jusqu'à 70°C, de préférence, 40°C à 60°C, puis on ajoute les proportions résiduelles des composants, en particulier, les proportions résiduelles des agents tensioactifs, l'électrolyte inorganique, le régulateur de pH, l'essence parfumante, le conservateur, ainsi que l'abrasif et on homogénéise le tout et on le refroidit finalement.

10. Procédé suivant la revendication 9, caractérisé en ce que l'on entreprend le mélange des composants et l'homogénéisation sans chauffage, dans le cas de l'emploi d'un alcool gras, à titre de composant.

11. Utilisation des agents de nettoyage de la peau suivant l'une quelconque des revendications 1 à 8, caractérisée en ce que, au cours du processus de nettoyage, on répartir l'agent de nettoyage, de préférence, d'abord sans addition d'eau, ou avec une faible proportion d'eau, sur la peau et on termine le processus de nettoyage sous rinçage avec de l'eau.
